# EUROPEAN PATENT APPLICATION

(11) **EP 3 967 366 A2**
(43) Date of publication of application: **16.03.2022**
(21) Application number: 21195738.6
(22) Date of filing: 09.09.2021
(51) Int. Cl.: A61N 5/10, A61B 6/00

(54) **APPARATUS FOR FAST CONE-BEAM TOMOGRAPHY AND EXTENDED SAD IMAGING IN RADIATION THERAPY**

(30) Priority: 11.09.2020 US 202017018244
(71) Applicant: Varian Medical Systems International AG, 6312 Steinhausen (CH)
(72) Inventor: MORF, Daniel, Buch am Irchel (CH); AMSTUTZ, Martin, CHAM (CH); KELLER, Simon, Villigen (CH)
(74) Representative: Foster, Mark Charles

(57) **Abstract**

A cone-beam computed tomography (CBCT) system employs a large area detector and a cone-beam radiation source to allow for acquisition of projection images in about 180 degrees plus a fan angle to provide a complete data set for CBCT reconstruction to generate an image volume with a field of view (FOV) of at least about 20 cm.

## Description

### TECHNICAL FIELD

This disclosure relates in general to radiation imaging and treatment. In particular, various embodiments of cone-beam computed tomography (CBCT) systems including large or extra-large image detectors and radiotherapy machines including the CBCT systems are described.

### BACKGROUND

Cone-beam computed tomography is known and has proved to be an invaluable tool for many clinical applications. In performing CBCT, a cone-beam radiation source and an image detector rotate about an object to acquire a plurality of projection images. The acquired projection images are reconstructed using computer software to generate tomographic images, which can be displayed or visualized in either two-dimensional (2D) image slices or three-dimensional (3D) image volumes of the object.

Image artifacts, distortions in reconstructed images, are common in CBCT. Some most severe image artifacts in CBCT are caused by patient motions or motions of internal anatomies. Such motions can happen randomly in time and on different anatomical locations. Some motions are periodic, some sporadic, and some just slow drifts. One approach to overcoming motion artifacts is to acquire projection images rapidly so that potential motions during acquisition can be minimized. This approach is favored in diagnostic CT. However, due to the system design of radiotherapy machines such as heavy gantries, rotation constraints, and collision avoidance, fast scanning as used in diagnostic CT has not been achieved on radiotherapy machines.

There are instances where a target volume cannot be placed in the isocenter of a radiotherapy machine, and a standard detector may be too small to cover the target volume when placed offset from the isocenter. One approach is to move the detector out laterally to image the target volume during treatment. This approach may help for treatment with a stationary gantry. For treatments with a rotating gantry, such as in volumetric modulated arc therapy (VMAT, RapidArc^{®}), intensity-modulated radiation therapy (IMRT), stereotactic radiosurgery (SRS), stereotactic body radiation therapy (SBRT) etc., the laterally offset approach may leave the target volume not visible or trackable for certain gantry angles.

Therefore, there is a general need for improving CBCT techniques. It would be desirable to provide a CBCT system for radiation therapy which allows for rapid data acquisition to reduce image artifacts. It would be desirable to provide a CBCT system for radiation therapy which allows for supervision or tracking of a target volume positioned offset from the isocenter of a radiotherapy machine at all gantry angles.

### SUMMARY

In a first aspect the present invention provides a system as defined in claim 1.

Optional features are defined in the dependent claims.

An example cone-beam computed tomography (CBCT) system is provided. The CBCT system comprises a source operable to produce a cone beam of radiation and an area detector operable to acquire a projection image of an object when irradiated with the cone beam of radiation. The source is rotatable about an axis and the area detector is rotatable in synchrony with the source. The area detector and the source are configured to allow for acquisition of a plurality of projection images in about 180 degrees plus a fan angle of the cone beam to provide a complete data set for CBCT reconstruction to generate an image volume with a field of view (FOV) of at least about 20 cm.

An example radiotherapy system is provided. The radiotherapy system comprises a first source rotatable about an axis and operable to produce radiation suitable for treatment, a second source operable to produce radiation suitable for imaging, and an area detector operable to acquire an image of an object when irradiated by the radiation from the second source. The area detector and the second source are rotatable in synchrony and configured to acquire an image of an object containing a reference volume located at least about 10 cm away from the axis at any angle of the first source.

An example system is provided. The system comprises a source operable to produce a cone beam of radiation and an area detector disposed opposite to the source operable to acquire a projection image of an object when irradiated with the cone beam of radiation. The area detector comprises a flat-panel imager having an active detection area comprising a first dimension ranging from 54 cm to 105 cm and a second dimension ranging from 43 cm to 86 cm.

This Summary is provided to introduce selected embodiments in a simplified form and is not intended to identify key features or essential characteristics of the claimed subject matter, nor is it intended to be used as an aid in determining the scope of the claimed subject matter. The selected embodiments are presented merely to provide the reader with a brief summary of certain forms the invention might take and are not intended to limit the scope of the invention. Other aspects and embodiments of the disclosure are described in the section of Detailed Description.

### BRIEF DESCRIPTION OF THE DRAWINGS

These and various other features and advantages of the disclosure will become better understood upon reading of the following detailed description and the appended claims in conjunction with the accompanying drawings, where:
FIG. 1 depicts an example radiation system according to embodiments of the disclosure.
FIG. 2 illustrates acquisition of projection images using a conventional CBCT system.
FIG. 3 illustrates acquisition of projection images using an example CBCT system according to embodiments of the disclosure.
FIG. 4A depicts a conventional radiotherapy machine equipped with a CBCT system including a detector positioned centrally on a beam axis.
FIG. 4B depicts a conventional radiotherapy machine equipped with a CBCT system including a detector positioned laterally offset from a beam axis.
FIGS. 5A-5D depict a radiotherapy machine equipped with a CBCT system according to embodiments of the disclosure. FIG. 5A shows an extended source to axis distance (SAD) application where the CBCT system carries out image acquisition while the radiation for treatment is delivered at a gantry angle of 0 degrees; FIG. 5B shows image acquisition at a gantry angle of 50 degrees; FIG. 5C shows image acquisition at a gantry angle of 170 degrees; and FIG. 5D shows image acquisition at a gantry angle of 240 degrees.

### DETAILED DESCRIPTION OF EMBODIMENTS

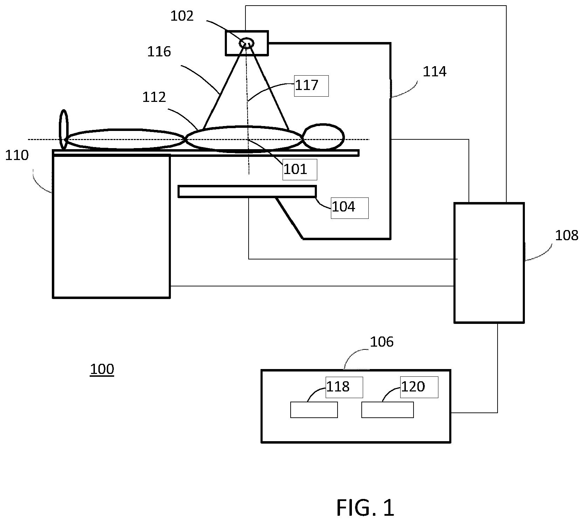

With reference to FIGS. 1-5D, various embodiments of a CBCT system and a radiotherapy machine including a CBCT system are described. In general, an example CBCT system comprises a cone-beam radiation source and an area detector operable to acquire projection images of a large object, or operable to acquire projection images encompassing a reference volume located away from the isocenter of the system. The area detector and the cone-beam radiation source are capable of acquiring projection images in about 180 degrees plus a fan angle, to provide a complete data set for CBCT reconstruction to generate an image volume with a field of view (FOV) of at least about 20 cm. Alternatively, or additionally, the area detector and the cone-beam radiation source are capable of acquiring projection images encompassing a reference volume located at about 10 cm or farther away from the isocenter of the system, at any gantry angle. As used in herein, the term "gantry angle," which may be used interchangeably with the term "angle of a radiation source," refers to an angle between a vertical line passing through the isocenter of a radiation system and a line passing through the isocenter and the radiation source.

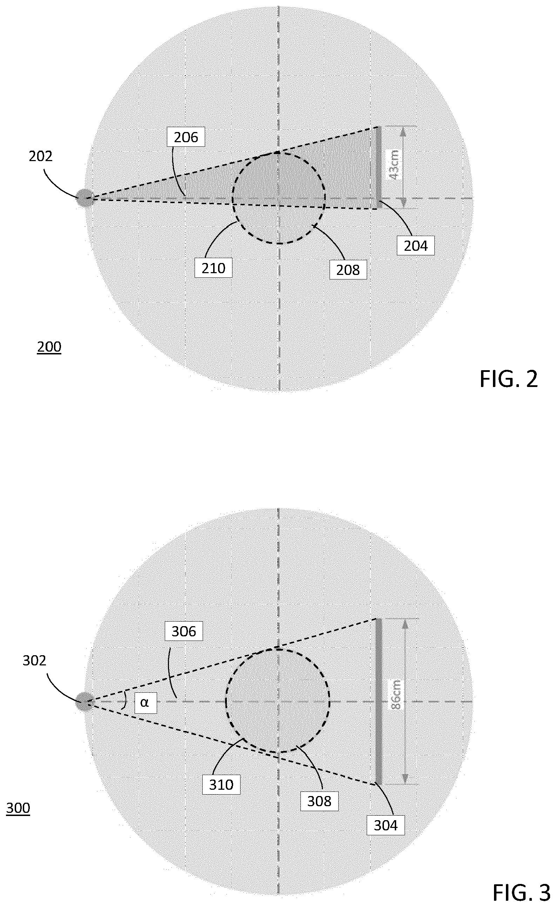

To image objects of large sizes e.g. a large anatomy in a patient, conventional radiotherapy machines equipped with a CBCT system need to rotate a detector and an imaging source in a full rotation (360 degrees) to acquire a complete data set needed for CBCT reconstruction. In the conventional imaging mode, the detector is offset with respect to the imaging source so that it can cover the whole anatomy in a full rotation, as shown in FIG. 2. According to embodiments of the disclosure, a large area detector and a cone-beam imaging source are used to cover the entire anatomy that needs to be scanned, as shown in FIG. 3. Because the area detector is sufficiently large, a half rotation plus a beam fan angle would be sufficient to acquire a complete data set for CBCT reconstruction. This cuts the acquisition time in about half and hence reduces the chance of patient and anatomy motions during image acquisition. As a result, image artifacts caused by patient or anatomy motions are minimized or significantly reduced. Any other optimization such as faster rotation of the source and detector can further enhance the imaging result. The optimization can bring the scan time in the range where breath hold techniques such as deep inspiration breath hold (DIBH) becomes possible. Applying DIBH techniques can further reduce patient motions and in some cases eliminate motions.

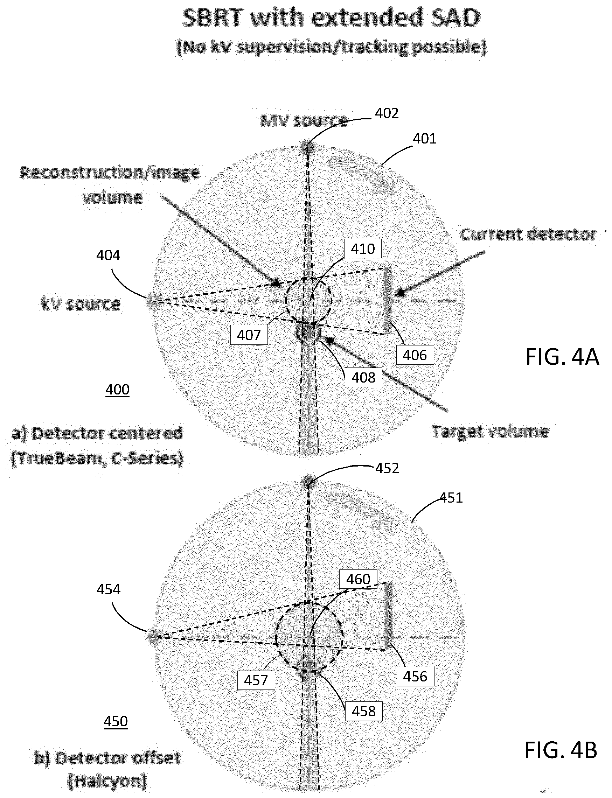

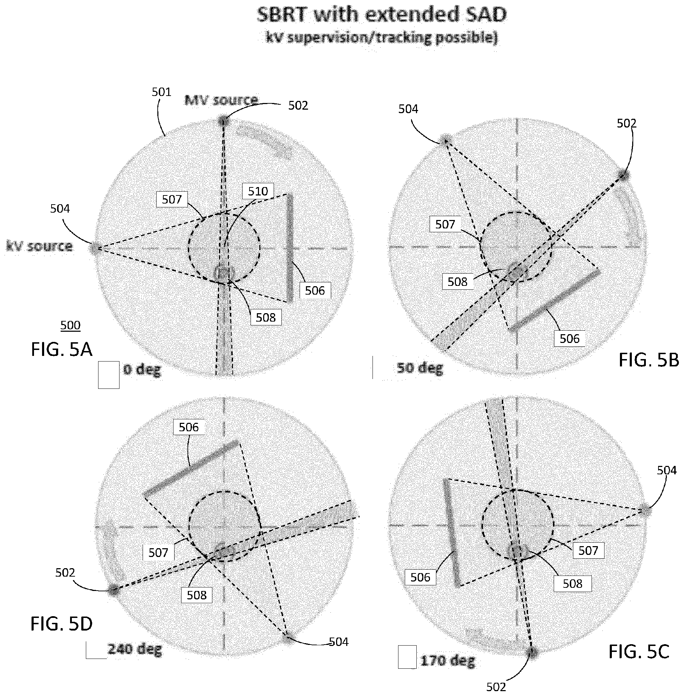

There are instances where a target volume is not placed in the isocenter of a radiotherapy machine. For stereotactic treatments such as SRS or SBRT, the radiation dose is delivered in high-dose fractions. It is important to ensure that the patient's anatomy does not move during the treatment. Motions of a target and organs at risk (OAR) are more likely to happen with a prolonged treatment time required for high-dose treatment. Any position error of the target or organs is more severe since the dose per fraction is much higher than in traditional therapy. It is therefore important to monitor the target, organs at risk, and reference structures or surrogates such as a diaphragm during the radiation exposure. In this disclosure, the term "reference volume" may be used to refer to a volume including a target such as tumorous tissue, organs at risk, reference structures or a surrogate as defined by a treatment planner. In a conventional radiotherapy machine equipped with a CBCT system, it is likely that the reference volume may not be in the field of view (FOV) when the detector is centered as shown in FIG. 4A. If the detector is positioned laterally offset, the reference volume will not be visible at certain gantry angles as shown in FIG. 4B. Advanced treatment techniques often use rotating gantry in order to optimize the dose distribution such as in VMAT, IMRT, etc. Therefore, the reference volume may not be visible during a large portion of the treatment. A radiotherapy machine equipped with a CBCT system according to embodiments of this disclosure can eliminate this problem such that a reference volume positioned away from the isocenter can be observed during the whole course of delivery of radiation for treatment as shown in FIGS. 5A-5D, to be described in greater detail below.

With reference to FIG. 1, an example CBCT system according to various embodiments of the disclosure is now described. It should be noted that the CBCT system of the disclosure can be implemented in a treatment system, a diagnostic system, a simulation system, a research and developmental system or any other suitable radiation system including an imaging functionality. For example, embodiments of the disclosure can be practiced in a radiotherapy machine which includes a radiation source operable at a megavoltage (MV) level e.g. 4 to 20 MV to produce radiation suitable for treatment of diseases. Therefore, embodiments of the disclosure may include a radiotherapy system adapted to perform intensity-modulated radiation therapy (IMRT) or volumetric modulated arc therapy (VMAT) where the treatment delivery is monitored, supervised, or guided with a CBCT system of the disclosure. Embodiments of the disclosure may also include a radiotherapy system adapted to perform stereotactic radiotherapy (SRS) or stereotactic body radiation therapy (SBRT) where a reference volume can be viewed and tracked at any gantry angles during the entire treatment. Embodiments of the CBCT system of the disclosure can also be used to help patient setup, patient verification, and treatment planning.

As shown in FIG. 1, an example CBCT system 100 in general includes a radiation source 102, an area detector 104, and an image processing device 106. The operation of the radiation source 102, the area detector 104, and the image processing device 106 can be controlled by a computer and control system 108. The computer and control system 108 may include one or more displays (not shown) to display a radiographic image, a fluoroscopic image, or a tomographic image generated by the system 100. A supporting device 110 e.g. a patient couch can place an object 112 e.g. a patient or a portion of the patient between the source 102 and the area detector 104 to receive radiation for imaging.

The source 102 is operable to produce a cone beam of radiation 116. As used herein, the term "cone beam" includes reference to a beam either conically shaped or pyramidally shaped. The shape and size of the cone beam 116 can be determined by various collimation devices such as collimator blocks, multileaf collimators (MLCs) etc. The cone beam 116 has an opening angle(s), which can be defined by a first fan angle in a first reference plane and a second fan angle in a second reference plane perpendicular to the first reference plane. By way of example, the two dimensions of a flat penal imager 104 can be used to reference the opening angles of the cone beam 116. As used in the disclosure, the term "fan angle" refers to an opening angle of a beam in a reference plane perpendicular to an axis about which the source producing the beam rotates. The cone beam 116 has a beam axis 117, a central beamline passing through the source 102 and the isocenter 101 of the system 100. The area detector 104 may be centrally positioned, aligning the center of the active detection area of the detector 104 to the beam axis 117. Alternatively, the area detector 104 may be positioned laterally offset from the beam axis 117.

The area detector 104 is operable to acquire projection images of the object 112 when irradiated with the cone beam 116. The area detector 104 may comprise a flat-panel imager including a plurality of detection elements arranged in rows and columns constituting an active detection area. An example detector 104 includes an active detection area of 54 x 43 cm². Another example detector 104 includes an active detection area of 86 x 43 cm². Another example detector 104 includes an active detection area of 86 x 65 cm². A further example detector 104 includes an active detection area of 105 x 86 cm². The detector 104 may include an active detection area having a dimension of about 54 cm or greater, or about 86 cm or greater, or a dimension ranging from about 54 to about 105 cm. The detector 104 may include an active detection area having a first dimension ranging from about 54 to about 105 cm and a second dimension ranging from about 43 to about 86 cm.

According to certain embodiments of the disclosure, the area detector 104 may be designed and fabricated as one single imager including a plurality of detection elements arranged in rows and columns. Alternatively, the area detector may be constructed by tiling or combining two or more sensor plates or panels of standard sizes available from various manufacturers. The two or more sensor plates or panels may be arranged next to each other or with some overlap and enclosed in a housing. In combining two or more sensor panels to construct an area detector, the tiling or overlapping region is preferably at an area away from the middle of the combined active detection area or away from the isocenter when positioned in the CBCT system. The artifacts caused by tiling or overlapping of the sensor panels can be corrected or calibrated by computer software.

Various area detectors and methods of making same are described in U.S. Application Ser. No. 15/978,924 filed May 14, 2018 entitled "Method for Fabricating Pixelated Scintillators." The disclosure of U.S. Application Ser. No. 15/978,924 is incorporated herein by reference in its entirety.

The area detector 104 and the source 102 can move or rotate in synchrony and acquire projection images of the object 112 from multiple angles. The area detector 104 and the source 102 can be supported or carried by a movable or rotating gantry 114. Alternatively, or additionally, the area detector 104 and the source 102 can be supported by separate arm structures respectively and move in synchrony. The gantry 114 may comprise a ring gantry, a C-arm gantry, or a robotic arm gantry operable to rotate the source 102 and the area detector 104 in synchrony. Alternatively, or additionally, the supporting device 110 can be moved in multiple degrees of freedom, e.g. translated horizontally and/or vertically, and/or rotated about various axes, to position the object 112 relative to the source 102 and area detector 104 before or during acquisition of projection images. In certain embodiments of the disclosure, the area detector 104 may be built in or attached to the supporting device e.g. a patient couch 110. This would be beneficial in radiographic imaging for diagnostic applications where a big field of view (FOV) is required for e.g. the spine, skeleton, total body, etc. Placing a large area detector in a couch can improve handling of the detector by eliminating or reducing the risk of damaging the detector 104 when being carried around, as currently is the case for many digital radiography systems. Instead of having the detector mechanically adjusted to the patient or the anatomy in the patient being imaged, the user can select a region of interest (ROI) on the area detector and image only the interested part of the body. In certain embodiments, the area detector 104 may be mounted on a stand to allow the patient to sit or stand during acquisition of projection images.

The image processing device 106 may include a computer 118 and software 120 designed to reconstruct tomographic images based on the projection images acquired by the area detector 104 and the source 102. Reconstruction software 120 is known in the art, including standard CBCT reconstruction software based on filtered back projection techniques and iterative CBCT (iCBCT) reconstruction software based on algebraic reconstruction techniques. For CBCT reconstruction, projection images of an object over a span of 180 degrees plus a beam fan angle are generally needed. A CBCT reconstruction volume or image volume refers to a volumetric space, generally in a cylindrical or spherical shape, over which tomographic images can be reconstructed based on the projection images. The diameter of a reconstruction volume is typically referred to as the field of view (FOV). In applications, an FOV of a reconstruction volume corresponds to or covers at least the size of the object that needs to be imaged. The FOV can be determined by the data in the projection images acquired by a CBCT system, which may be generally determined by the size of the area detector and the cone beam of the CBCT system where the distance between the source and the area detector is predetermined.

According to certain embodiments of the disclosure, the area detector 104 and the cone-beam source 102 are configured to acquire projection images of a complete data set for CBCT reconstruction in about 180 degrees plus a beam fan angle, to provide an image volume with an FOV of at least about 20 cm or greater. This allows for rapid acquisition of a data set to reduce image artifacts. A large anatomy may have a size of about 20 cm or greater, or about 30 cm or greater, or about 35 cm or greater, or about 40 cm or greater, or ranging from about 20 cm to about 50 cm. According to certain embodiments of the disclosure, the area detector 104 and the cone-beam source 102 can be configured to acquire projection images to provide a complete data set for CBCT reconstruction in half a rotation plus a beam fan angle, to generate an image volume with an FOV of about 20 cm or greater, or about 30 cm or greater, or about 35 cm or greater, or about 40 cm or greater, or with an FOV ranging from about 20 to about 50 cm. This contrasts to conventional CBCT systems with which the maximal size of an object that can be covered is generally about or less than 20 cm, wherein a complete data set for CBCT reconstruction is acquired in half a rotation plus a fan angle. Alternatively, or additionally, according to certain embodiments of the disclosure, the area detector 104 and the cone-beam source 102 can be configured to acquire a projection image that covers a reference volume placed away from the isocenter about 10 cm or farther, or about 15 cm or farther, or about 17.5 cm or farther, or about 20 cm or farther, at any gantry angle in 360 degrees, or covers a reference volume placed away from the isocenter from about 10 to about 25 cm at any gantry angle.

With reference now to FIGS. 2-3, the CBCT system according to embodiments of the disclosure allows for fast acquisition of projection images of large objects for CBCT reconstruction. For purpose of comparison, FIG. 2 illustrates acquisition of projection images of a large object using a conventional CBCT system 200. The conventional CBCT system 200 includes a kV source 202 and an image detector 204 having a size of 43 x 43 cm². The image detector 204 is positioned laterally offset from the beam axis 206 and distanced from the kV source 202 at 150 cm. To acquire projection images of a large object 208 (e.g. about 50 cm in a dimension) for CBCT reconstruction, the image detector 204 and the kV source 202 start the scan from a periphery portion of the object 208. The object 208 must be scanned in 360 degrees to acquire a complete data set for CBCT reconstruction. Reference 210 indicates an image volume reconstructed based on the projection images of the object 208 acquired by the CBCT system of 200. A full rotation in 360 degrees requires longer time, which increases the chance of patient motions, and thus the possibilities of causing image artifacts.

FIG. 3 illustrates acquisition of projection images of a large object using an example CBCT system 300 according to embodiments of the disclosure. The example CBCT system 300 comprises a kV source 302 and a large image detector 304 having a size e.g. 86 x 43 cm². The image detector 304 is centrally positioned on the beam axis 306 and distanced from the kV source 202 at about 150 cm. To acquire projection images of the object 308 (e.g. about 50 cm diameter) for CBCT reconstruction, a scan of the object 308 in about 180 degrees plus a fan angle (alpha) would be sufficient to acquire a complete data set for CBCT reconstruction. Reference 310 indicates an image volume reconstructed based on the projection images of the object 308 acquired by the CBCT system 300 of this disclosure. The capability of acquiring a complete data set of large objects in about half a rotation plus a fan angle significantly cuts the acquisition time, reduces the chance of patient motions during image acquisition, and thus reduces or eliminates image artifacts caused by patient motions.

The capability of rapidly acquiring a complete data set of large objects for CBCT reconstruction in about half a rotation plus a beam fan angle enables the use of deep inspiration breath hold (DIBH) techniques, which can further help reduce the chance of patient motions, and thus reduce or eliminates image artifacts caused by patient motions. DIBH is a radiation therapy technique where patients take a deep breath during treatment, and hold this breath while the radiation is delivered. In a radiotherapy machine equipped with the CBCT system of the disclosure, the operation of the machine can be optimized to allow the gantry to rotate at a relatively fast speed, e.g. four revolutions per minute. At this rotating speed, it would take the CBCT system of the disclosure about 7-8 seconds to acquire a complete data set in a scan of about 180 degrees plus a fan angle while allowing the bigger FOV of up to 50 cm and greater. The 7-8 second acquisition time is within the range for application of DIBH techniques because most people can hold the breath for 6-9 seconds.

The capability of rapid acquisition of projection images for CBCT reconstruction allows for better use of the imaging source 302 e.g. an x-ray tube. For a given image quality, only half of the total x-ray tube power is required for the CBCT system of the disclosure as compared to conventional systems. Generation of x-rays is isotropic controlled by physics. Nevertheless, the x-ray beam must be collimated to irradiate an object onto the active detection area of an image detector. Therefore, all the generated radiation that does not hit the image detector is wasted. A large detector can make more efficient use of the generated x-rays because it provides a large active detection area. Generation of x-rays is a very inefficient process, where 99% of the required energy can be wasted in the form of heat. The CBCT system of the disclosure with a large detector (e.g. double the size of the conventional imager) can provide double of the dose for the same heat dissipation. This would allow to select a smaller x-ray tube, thus reduce the cost for comparable image quality.

The CBCT system 300 of the disclosure also reduces the mechanical complexity of the structure supporting the detector 304. Because no lateral movement would be required to move the detector 304 laterally out of the beam axis, the detector 304 can be mounted on a robotic wrist without the need for a lateral movement mechanism.

With reference to FIGS. 4A-4B and 5A-5D, embodiments of the disclosure allow for supervision or tracking of a reference volume during radiation treatment in extended source to axis distance (SAD) applications. There are instances where a reference volume is not placed in the isocenter of a radiotherapy system, and a conventional standard image detector may be too small to cover the reference volume when located away from the isocenter. For example, due to the patient anatomy, the reference volume may have to be placed away from the isocenter. The clearance between the patient body surface and the treatment head of the radiotherapy machine may be insufficient for some patients. Some treatments may require constant source to skin distance (SSD). Further, it has proved that VMAT (RapidArc^{®}) treatment with "couch kicks" (rotation of the patient couch) can spare out organs at risk and allow for better dose deposition in the target. The small couch kick allowed in conventional treatment systems limits the gantry rotation angle due to the potential risk of collisions. Therefore, moving the reference volume e.g. in the range of additional 5-15 cm or even 20 cm away from the isocenter would significantly help in reducing the risk of collisions and safely enable VMAT (RapidArc^{®}) with couch kicks.

FIG. 4A depicts a conventional radiotherapy system 400 in an extended SAD application. The conventional radiotherapy system 400 comprises an MV source 402 producing therapeutic radiation for treatment, a kV source 404, and a detector 406. A reference volume 408 is located offset from the isocenter 410 of the radiotherapy system 400 for reasons discussed above. A C-arm gantry 401 carries the MV source 402, allowing delivery of therapeutic radiation to tumorous tissue in the reference volume 408 while the MV source 402 is rotating about an axis passing through the isocenter 410, as indicated by the arrow, to perform SRS, SBRT, or VMAT. The kV source 404 and the image detector 406 are also supported by the rotating gantry 401 e.g. via extended arms (not shown), and operate to acquire images such as planar images, radiographic images, or fluoroscopic images for monitoring, supervising, or tracking the reference volume 408 during the treatment. In FIG. 4A, the detector 406 is centrally positioned on the beam axis of the kV source 404. Reference 407 indicates an FOV of the detector 406 at any gantry angle. As shown in FIG. 4A, the reference volume 408 located offset from the isocenter 410 falls outside the FOV 407 and cannot be imaged by the detector 406 at certain gantry angles. As such, the reference volume 408 cannot be supervised or tracked by the detector 406 equipped on the radiotherapy system 400 during the entire treatment.

FIG. 4B depicts another conventional radiotherapy system 450 in an extended SAD application. The conventional radiotherapy system 450 comprises an MV source 452 producing therapeutic radiation for treatment, a kV source 454, and a detector 456. A reference volume 458 is located offset from the isocenter 460 of the radiotherapy system 450 for reasons discussed above. In comparison with the radiotherapy system 400 of FIG. 4A, the radiotherapy system 450 in FIG. 4B includes a ring gantry 451 carrying the MV source 452, the kV source 454, and the detector 456. The detector 456 is positioned laterally offset from the beam axis of the kV source 454. Reference 457 indicates a volume that can be imaged by the detector 456 at certain gantry angles during a full rotation. As shown in FIG. 4B, a portion of the reference volume 458 located offset from the isocenter 460 falls outside the volume 457, and thus cannot be supervised or tracked by the detector 456 equipped on the radiotherapy system 450 during the entire treatment. For stereotactic treatments such as SRS or SBRT, the radiation is delivered in fewer high-dose fractions and it is important to monitor the target volume during the entire radiation exposure. Any position error of the target volume is more severe because the dose per fraction is much higher.

FIGS. 5A-5D depict a radiotherapy system 500 according to embodiments of the disclosure in an extended SAD application. The radiotherapy system 500 comprises an MV source 502, a kV source 504, and an area detector 506. A reference volume 508 is located offset from the isocenter 510 of the radiotherapy system 500 for reasons discussed above, e.g. 25 cm away from the isocenter 510. A rotating gantry 501 carries the MV source 502, allowing delivery of therapeutic radiation to tumorous tissue in the reference volume 508 while the MV source 502 is rotating about an axis passing the isocenter 510, as indicated by the arrow, to perform SRS, SBRT, or VMAT. The rotating gantry 501 can be a C-arm gantry, a ring gantry, or a robotic gantry. The detector 506 and the kV source 504 may be supported by the gantry 501 e.g. via extended arms (not shown), and operate to acquire images from a plurality of angels as the gantry 501 rotates. Alternatively, the detector 506 and the kV source 504 may be supported by a separate gantry. In the embodiments shown in FIGS. 5A-5D, the detector 506 is centrally positioned on the beam axis of the kV source 504. As shown, images encompassing the reference volume 508 can be acquired by the detector 506 and the kV source 504 at any gantry angle of the MV source 502, e.g. at 0 degree shown in FIG. 5A, at 50 degrees shown in FIG. 5B, at 170 degree shown in FIG. 4C, and at 240 degree shown in FIG. 5D. The reference volume 508, while being positioned e.g. 25 cm away from the isocenter 510, remains in the FOV of the image detector 506, allowing supervision and tracking of the reference volume 508 at any gantry angle of the MV source 502 in 360 degrees.

According to some embodiments of the disclosure, the kV source 504 can be synchronized with the MV source 502 to allow acquisition of images in sync with a pause pattern of treatment delivery to the target. For instance, the radiation from the MV source 502 can be paused for a short period of time to allow the kV source 504 to deliver kV beam and acquire an image. This synchronization can reduce the effect of creating artifacts on the acquired image which otherwise could be caused by scattering of the treatment beam from the MV source 502. By way of example, the treatment beam from the MV source 502 can be paused in 100 ms to allow the kV source 504 to deliver kV beam and the detector 506 to readout image signals during that pause. Alternatively, or additionally, the detector 506 can be synchronized with the MV source 502 to allow the readout of the detector 506 in sync with a pulse pattern of the treatment beam from the MV source 502. By way of example, the treatment beam from the MV source 502 can be delivered in short e.g. 5 µs pulses in every 2.5 ms, and the line readout of the detector 506 can be carried out in sync with the treatment beam pulses. This would allow artifacts caused by scattering of the treatment beam from the MV source 502 to appear at the same location in the detector 506, which can be corrected at least to some extent.

Returning to FIG. 1, the radiation source 102 may be a source operable to produce photons, protons or other heavy ions, and electrons. By way of example, the source 102 may include an x-ray tube comprising a target which generates x-rays upon impingement of electrons. The x-ray tube 102 may include a collimator device to collimate the beam so that the beam exiting the x-ray tube generally in a conical or pyramidal shape. As another example, the source 102 may include a linear accelerator comprising a target which generates x-rays upon impingement of electrons, and various collimation devices for shaping and/or sizing the x-rays. As a further example, the source 102 may include a nozzle emitting protons produced by a cyclotron or synchrotron and transported to the nozzle. In general, the source 102 may produce or emit radiation suitable for imaging. For example, the source may be operated at a kilovoltage (kV) level e.g. ranging from 50 to 150 kV to produce radiation suitable for diagnostic imaging. Alternatively, the source may be operated at a megavoltage (MV) level for use with e.g. an electronic portal imaging device (EPID) to acquire images e.g. for determining patient setup or for interventional treatment or treatment plan iteration. In some embodiments, the source 102 may be a source operable at two or multiple modes, e.g. at either kV or KV energy levels to produce radiation for various applications including dual-energy or multi-energy imaging.

In a specific embodiment, the source 102 includes an x-ray tube operable to produce x-rays. X-ray tubes are well known in the art and therefore their detailed description is omitted herein. Briefly and in general, an x-ray tube includes a source of electrons (cathode) e.g. a filament and a target e.g. a tungsten metal on a rotating plate (anode) enclosed in an evacuated envelope. A supply of current causes the filament to be heated producing electrons. A voltage applied to the evacuated envelope accelerates the electrons towards the anode striking the target to produce x-rays. The small surface area on the target producing the x-rays is referred to as the focal spot of the x-ray tube or source, the size of which may be determined by the electron beam from the cathode. The x-rays produced can be then shaped by a collimator and exit through a window in the envelope. While not shown in FIG. 1, an x-ray controller and generator set signals such as current, voltage, exposure time, and other operating parameters of the x-ray tube, and generates a voltage to be supplied to the x-ray tube. The x-ray controller and generator in turn can be controlled by the computer and control system 108.

Still with reference to FIG. 1, the area detector 104 detects the radiation transmitted through the object 112, which is indicative of the attenuating properties or the structure of the imaging portion of the object 112. The area detector 104 may be coupled with a data acquisition system (not shown), which includes electronic circuitries for providing control signals, receiving and processing data signals, and outputting data to the image processing device 106. The data acquisition system and the area detector 104 can be separate units or integrated as a single unit controlled by the computer and control system 108.

The area detector 104 comprises a plurality of detection elements arranged in rows and columns in a two-dimensional area. In a specific embodiment, the area detector 104 includes a flat-panel imager having a flat detector surface. In an alternative embodiment, the area detector 104 has a curved detector surface.

In certain embodiments, the area detector 104 may include or may be used in conjunction with an anti-scatter grid. Because the image detector has a sufficiently large active detection area, a two-dimensional grid can be used to reduce scatter from both directions and no movement of the area detector with respect to the grid is required even for larger objects. The anti-scatter grid can be focused or non-focused. Various embodiments of anti-scatter grids are described in U.S. Pat. No. 9,620,256 entitled to "X-Ray Imaging Device Including Anti-Scatter Grid," assigned to Varian Medical Systems, Inc. The disclosure of U.S. Pat. No. 9,620,256 is incorporated herein by reference in its entirety.

In certain embodiments, the area detector 104 may be a multi-layer image detector. A multi-layer image detector includes two or more detection layers arranged one on top of the other, or two or more imagers arranged one on top of the other. A multi-layer image detector can increase the overall dose efficiency and allow for use dual-energy imaging techniques. For example, a detection layer proximal to the source can be configured to read the low energy portion of the image signal and a detection layer distal to the source can be configured to read the high energy content of the image signal. By clever processing of these sub-images, more information of the imaged anatomy can be obtained such as the density, Z-values, electron density, etc. CBCT reconstruction can be conducted for virtual monoenergetic beam energies. Various embodiments of multi-layer image detectors are described in U.S. Pat. No. 9,268,037 entitled to "Universal kV-MV Imagers," assigned to Varian Medical Systems, Inc. The disclosure of U.S. Pat. No. 9,268,037 is incorporated herein by reference in its entirety.

In certain embodiments of the disclosure, the area detector 104 may be a flat-panel imager. The flat-panel imager may include a radiation conversion layer and a detector array. The conversion layer converts radiation such as x-ray photons to visible light. The detector array detects the visible light and converts it to electrical signals. The conversion layer may include a scintillator material which can generate visible light photons in response to x-ray radiation. As such, the detector array may include photosensitive elements which can generate electrical signals in response to the light photons generated by the scintillator material. Suitable scintillator materials include gadolinium oxysulfide (Gd₂O₂S:Tb), cadmium tungstate (CdWO₄), bismuth germinate (BiₐGe₃O₁₂ or BGO), cesium iodide (Csl), cesium iodide thallium (Csl:TI), Thallium doped Sodium Iodide Nal(TI) or any combination thereof. Suitable photosensitive element may include a photodiode, a photogate, or phototransistors etc. Alternatively, the conversion layer may include a photoconductor material which can convert x-ray photons to electrical charges (electron-hole pairs) directly. As such, the detector array may include electrodes at either side of the photoconductor material to collect the electrical charges generated by the photoconductor material. Suitable photoconductor materials include and are not limited to mercuric iodide (Hgl₂), lead iodide (Pbl₂), bismuth iodide (Bil₃), cadmium zinc telluride (CdZnTe), amorphous selenium (a-Se), etc.

In certain embodiment, an example flat-panel imager 104 may include a large number e.g. hundreds of thousands or millions of detector pixels. The large number of detector pixels may be arranged in a plurality of rows and a plurality of columns forming an active detector area. In some specific embodiments, each detector pixel may include an addressable photosensitive element such as a photodiode and a switching transistor such as a thin-film transistor (TFT) or complementary metal oxide semiconductor (CMOS) transistor.

The detector array may further include a plurality of address lines and a plurality of data lines. Each of the plurality of address lines may connect a plurality of detector pixels in a row to a driver control assembly of the data acquisition system. Each of the plurality of data lines may connect a plurality of detector pixels in a column to a readout control assembly of the data acquisition system. The driver control assembly provides control signals for accessing a selected row of pixels. The readout control assembly provides control signals for reading out the signals from the pixels. By way of example, when it is desired to capture image signals from the detector array, a control signal from the driver control assembly drives the gates of switching elements e.g. TFTs in a selected row of pixels, and the signals stored in the selected row of pixels are readout by the readout control assembly. The signals from the selected pixels may be buffered, amplified, and converted by analog-to digital converters (ADCs) using electronics of the data acquisition system. The resulting digitized data signals can be then multiplexed, buffered, and transmitted to an image processing device for further processing.

In operation of the detector arrays, the control signal from the driver control circuit for a row of pixels can be asserted on an address line for a predetermined period of time or line time. During assertion of the control signal, the signal from each pixel in the selected row is transmitted via column data lines to the readout control circuit where the signals on each data line are received and buffered by a corresponding charge sensitive amplifier. Hence, an entire row of image data can be captured in a line time period. With each subsequent line time period, a subsequent row of image data is captured. At the end of a "frame time" period, the entire image can be captured. In this manner, the pixels contained in the entire active detection area can be read out, row-by-row, each row in a line time period. In an alternative embodiment, the flat-panel imager may employ a split data-line where the top half of the array and the bottom half of the array are read out simultaneously. This allows the flat-panel imager to read out more rapidly, e.g., only half of the "frame time" is needed to read out the pixels. By way of example, the flat-panel imager can operate at a speed ranging from 15 to 50 frames per second (fps), depending on the dose requirements, image resolution, and the number of projection data sets acquired.

Still with reference to FIG. 1, the image processing device may include a computer 118 and software 120 designed to process the projection images and reconstruct tomographic images based on the projection images. The computer 118 may comprise a processor, memory, optionally a user interface, and a network interface. The processor may include a central processing unit (CPU) that is generally known in the art, such as an INTEL^{®} processor or an AMD^{®} processor, or a graphical processing unit (GPU), such as an NVIDIA^{®} GPU, or other type of processing unit. The processor may retrieve and execute computer-executable instructions from the memory, which may cause the processor to perform any of the methods and/or steps according to the embodiments of this disclosure described above.

The memory may include any one of or a combination of volatile memory elements and nonvolatile memory elements. The memory may include a random-access memory (RAM) or other dynamic storage device for storing information and instructions to be executed by the processor, and for storing temporary variables or other intermediate information during execution of instructions by the processor. The memory may also include read-only memory (ROM) or other static storage device for storing static information and instructions for the processor. The memory may further include a data storage device such as a magnetic disk or optical disk, for storing information and instructions. The memory (e.g. a non-transitory computer-readable medium) may comprise programs (logic) for operating the computer system and for performing applications including dosimetric projection and dose calculation as described above, or other treatment planning applications. In addition, the memory may include a database storing any information that can be selected by a user, such as a radiation oncologist or radiation therapist.

Various embodiments of CBCT systems and radiotherapy machines including the CBCT systems are described with reference to the figures. It should be noted that some figures are not necessarily drawn to scale. The figures are only intended to facilitate the description of specific embodiments and are not intended as an exhaustive description or as a limitation on the scope of the disclosure.

All technical and scientific terms used herein have the meaning as commonly understood by one of ordinary skill in the art unless specifically defined otherwise. As used in the description and appended claims, the singular forms of "a," "an," and "the" include plural references unless the context clearly dictates otherwise. The term "or" refers to a nonexclusive "or" unless the context clearly dictates otherwise. The term "first" or "second" is used to distinguish one element from another in describing various similar elements and should not be construed as in any particular order unless the context clearly dictates otherwise. Further, certain specific dimensions are provided for thorough understanding of various aspects of the disclosure. It should be noted that these specific dimensions are not intended to limit the scope of the claims. The specified dimensions or parameters should be construed to mean "about," "approximate" rather than "perfect." For example, the term "about" may be used to indicate a value that can include a variation of ± 15% of the value modified by the term.

Those skilled in the art will appreciate that various other modifications may be made. All these or other variations and modifications are contemplated by the inventors and within the scope of the invention.

## Claims

1. A system, comprising:
a source operable to produce a cone beam of radiation, the source being rotatable about an axis to irradiate an object from a plurality of angles;
an area detector rotatable in synchrony with the source, the area detector being operable to acquire a projection image of the object when irradiated with the cone beam of radiation, wherein the area detector and the source are configured to allow for acquisition of a plurality of projection images in about 180 degrees plus a fan angle of the cone beam to provide a complete data set for CBCT reconstruction to generate an image volume with a field of view (FOV) of at least about 20 cm.

2. The system of claim 1, wherein the area detector and the source are configured to allow for acquisition of the plurality of projection images in about 180 degrees plus the fan angle to provide the complete data set for CBCT reconstruction to generate the image volume with an FOV ranging about from 20 to 50 cm.

3. The system of claim 1 or claim 2, wherein the area detector is positioned centrally on a beam axis of the cone beam of radiation.

4. The system of any preceding claim, further comprising:
a source operable to produce radiation suitable for treatment of a disease and rotatable about the axis, the source operable to produce radiation suitable for treatment, optionally, being rotatable about the axis at a speed of at least one revolution per minute.

5. The system of any preceding claim, wherein the source is rotatable about the axis to irradiate a patient containing the object, and the area detector and the source are configured to acquire the plurality of projection images in about 180 degrees plus a fan angle of the cone beam while the patient is holding breath.

6. The system of any preceding claim, wherein the area detector comprises a flat-panel imager having an active detection area comprising a dimension ranging from about 54 cm to about 105 cm.

7. The system of claim 6, wherein the area detector comprises two or more sensor plates or sensor panels forming the active detection area.

8. A system, comprising:
a first source rotatable about an axis and operable to produce radiation suitable for treatment;
a second source operable to produce radiation suitable for imaging; and
an area detector operable to acquire an image of an object when irradiated by the radiation from the second source, the object containing a reference volume located at least about 10 cm away from the axis and including a target to be irradiated by the radiation from the first source, the area detector being rotatable in synchrony with the second source, wherein the area detector and the second source are configured to allow for acquisition of the image encompassing the reference volume at any angle of the first source.

9. The system of claim 8, wherein the reference volume in the object is located at about 10-25 cm away from the axis, and the area detector and the second source are configured to allow for acquisition of the image encompassing the reference volume at any angle of the first source.

10. The system of claim 8 or claim 9, wherein the first source is configured to produce the radiation suitable for stereotactic radiosurgery (SRS) or stereotactic body radiation therapy (SBRT);
and/or
wherein the area detector is positioned centrally on a beam axis of the radiation.

11. The system of any of claim 8 to claim 10, wherein the area detector comprises a flat-panel imager having an active detection area comprising a dimension ranging from about 54 cm to about 105 cm.

12. The system of any of claim 8 to claim 11, wherein the second source is synchronized with the first source to allow the second source to deliver radiation and acquire the image of the object while the first source is paused, thereby reducing an effect of creating an artifact on the image caused by scattering of the radiation from the first source;
and/or
wherein the area detector is synchronized with the first source to allow the area detector to acquire the image of the object in synchrony with a pulse pattern of the first source.

13. The system of any of claim 8 to claim 12, wherein the area detector comprises two or more detection layers configured for dual-energy or multi-energy imaging;
or
wherein the area detector comprises a flat-panel imager having an active detection area formed by two or more sensor plates or sensor panels.

14. A system, comprising:
a source operable to produce a cone beam of radiation; and
an area detector disposed opposite to the source operable to acquire a projection image of an object when irradiated with the cone beam of radiation, wherein the area detector comprises a flat-panel imager having an active detection area comprising a first dimension ranging from about 54 cm to about 105 cm and a second dimension ranging from about 43 cm to about 86 cm.

15. The system of claim 14, further comprising a couch supporting a patient, wherein the area detector is mounted in the couch;
and/or
wherein the area detector comprises a flat-panel imager having an active detection area formed by two or more sensor plates or sensor panels.
